(19) Europäisches Patentamt
European Patent Office
Office européen des brevets

(11) EP 3 355 868 B1

(12) **FASCICULE DE BREVET EUROPEEN**

(45) Date de publication et mention de la délivrance du brevet:
**11.03.2020 Bulletin 2020/11**

(21) Numéro de dépôt: **16775634.5**

(22) Date de dépôt: **27.09.2016**

(51) Int Cl.:
*A61K 9/46* (2006.01) *A61K 9/12* (2006.01)
*A61K 31/573* (2006.01) *A61P 17/06* (2006.01)

(86) Numéro de dépôt international:
**PCT/EP2016/073015**

(87) Numéro de publication internationale:
**WO 2017/055298 (06.04.2017 Gazette 2017/14)**

(54) **MOUSSE CHIMIQUE NON RINCÉE CONTENANT DU PROPIONATE DE CLOBÉTASOL, ET SON UTILISATION DANS LE TRAITEMENT DU PSORIASIS**

CHEMISCHER SCHAUM OHNE AUSSPÜLEN MIT CLOBETASOLPROPIONAT UND VERWENDUNG DAVON BEI DER BEHANDLUNG VON PSORIASIS

NO-RINSE CHEMICAL FOAM CONTAINING CLOBETASOL PROPIONATE, AND USE THEREOF IN THE TREATMENT OF PSORIASIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.09.2015 FR 1559203**

(43) Date de publication de la demande:
**08.08.2018 Bulletin 2018/32**

(73) Titulaire: **Galderma Research & Development
06410 Biot (FR)**

(72) Inventeurs:
- **BUGE, Jean-Christophe
06200 Nice (FR)**
- **NADAU-FOURCADE, Karine
06270 Villeneuve Loubet (FR)**

(74) Mandataire: **Casalonga
Casalonga & Partners
Bayerstraße 71/73
80335 München (DE)**

(56) Documents cités:
**WO-A1-00/27356** **WO-A1-2014/201541**
**US-A1- 2004 184 992** **US-A1- 2013 244 976**

EP 3 355 868 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet un produit topique non rincé sous forme d'une mousse pour le traitement pharmaceutique de la peau, et plus particulièrement du psoriasis, et qui contient du propionate de clobétasol.

Le psoriasis est une des maladies de la peau les plus fréquentes parmi toutes les maladies chroniques de la peau. Ce dernier provoque essentiellement des érythèmes, desquamations, hyperkeratoses ou prurits et est la source d'un fort inconfort pour les personnes atteintes de cette maladie. Les traitements utilisés à ce jour reposent principalement sur l'application de crèmes et d'onguents sur les plaques. Ils contiennent de l'acide salicylique, des stéroïdes locaux, du goudron. Ces traitements sont déplaisants, notamment l'application de goudron, et nécessitent de longues applications.

Le propionate de clobétasol (Clobetasol propionate is 21-chloro-9-fluoro-11β,17-dihydroxy-16β-methylpregna-1,4diene-3, 20-dione 17-propionate) est un dermocorticoïde de classe 1, à savoir un corticoïde de très forte activité. Comme les autres dérivés de la cortisone à usage local, il freine le renouvellement et la multiplication des cellules de la peau. Son efficacité sur le psoriasis est avérée.

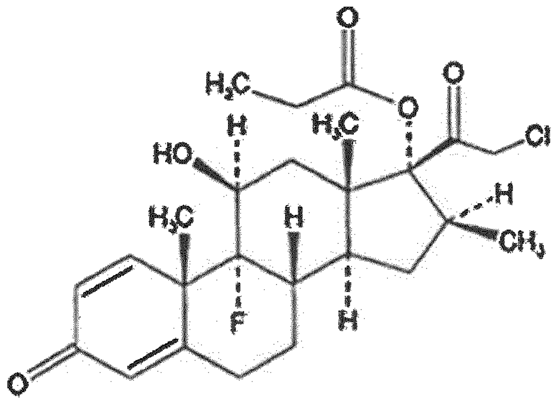

Il existe le besoin de disposer de nouvelles formes galéniques pour une application topique dans lesquelles le propionate de clobétasol est stable, bien toléré, efficace et agréable à appliquer.

L'application de crèmes et pommades est indispensable dans le traitement du psoriasis. Ces soins locaux visent à diminuer les squames et améliorer le confort cutané et l'aspect de la peau. Ces traitements comprennent des crèmes hydratantes simples et/ou des crèmes dans lesquelles sont ajoutées des agents qui vont permettre de mieux détacher les squames et que l'on appelle des kératolytiques. Dans tous les cas les traitements doivent apporter beaucoup d'émollience pour assouplir la peau.
Dans les traitements pharmaceutiques de ces pathologies, des produits sont donc appliqués sur de très larges surfaces, et sur des peaux qui peuvent être fragilisées mais aussi épaissies et difficiles à pénétrer.

Ainsi, Il existe donc le besoin de disposer de nouvelles formes galéniques et en particulier de type mousses ou compositions moussantes permettant un meilleur contrôle de la dose, une couvrance importante, et dans lesquelles le propionate de clobétasol est stable, bien toléré, efficace et agréable à appliquer, notamment afin d'améliorer l'observance du traitement.

La composition selon l'invention présente l'avantage d'être sous forme d'une mousse générée de façon extemporanée et très bien tolérée.

Après son application, la composition selon l'invention n'est pas éliminée par rinçage.

Un des avantages de la composition est d'être particulièrement bien tolérée, malgré le fait qu'elle ne soit pas éliminée par rinçage, comme le montrent les exemples illustrant une des méthodes d'évaluation de la tolérance présentés ci-après.

Il existe différentes méthodes d'évaluation de la tolérance d'un produit pharmaceutique ou cosmétique à usage cutané parmi lesquels on peut citer le test in vivo « in used » or « human patch test » mais aussi le test in vitro tel que le test de mesure de l'irritation sur Epiderme Humain Reconstruit (Reconstructed Human Epidermis ou RHE) décrit dans le protocole TG439 OCDE. Cette dernière méthode est détaillée dans l'exemple 3.

Il existe actuellement des mousses ou compositions moussantes sur le marché. Toutefois, elles présentent toutes un certain nombre d'inconvénients :
En effet, il existe 3 types de mousses ou compositions moussantes :

- Des aérosols dans lesquels la mousse est générée par un gaz propulseur mais avec l'inconvénient d'être des aérosols présentant les risques bien connus de ceux-ci (pollution, risques respiratoires notamment).

- Des crèmes foisonnées dans lesquelles des bulles d'air sont introduites dans le produit par l'intermédiaire d'un procédé de fabrication particulier. Ce procédé présente l'inconvénient d'être contraignant au niveau industriel et nécessite un lourd investissement au niveau du matériel de conditionnement.
- Des formules moussantes contenant des tensioactifs moussants causant généralement des problèmes d'irritation provenant des propriétés détergentes de ces tensioactifs moussants.

Ainsi, il subsiste donc le besoin de mettre au point une composition pharmaceutique dont la forme galénique est différente des formes galéniques connues afin, entre autres, de permettre de disposer de compositions destinées à une application topique contenant du propionate de clobétasol dans des compositions bien tolérées et donc de remédier au problème d'irritation, destinées à une application topique chez l'être humain, en particulier une application non rincée (c'est-à-dire que la composition n'est pas éliminée par rinçage après son application).

Le but de la présente invention est donc de proposer une composition répondant à ces besoins.

La demanderesse a ainsi mis au point une nouvelle composition pharmaceutique, destinée à une application topique non rincée qui se présente sous la forme d'une mousse qui ne contient avantageusement pas de tensioactif moussant. On définit par tensioactif moussant, des tensioactifs qui produisent une mousse volumineuse, stable et onctueuse lorsqu'ils sont mélangés à l'eau selon les tests bien connus de l'homme du métier.

Constituent des tensioactifs moussants : les tensioactifs anioniques, les tensioactifs cationiques, les tensioactifs amphotères et les tensioactifs non ioniques de la famille des alkylpolyglucosides et des glucamides.

La forme galénique selon l'invention présente l'avantage de garantir une bonne stabilité du propionate de clobétasol. De plus, cette formulation conduit avantageusement à l'obtention d'une mousse douce, parfaitement tolérée et non irritante, qui permet une meilleure couverture de la zone à traiter et permet de pallier les problèmes de tolérance par un meilleur contrôle de la dose, grâce aux propriétés d'étalement et la faible densité de la mousse.

Enfin, de manière avantageuse, cette forme galénique ne nécessite pas pour sa mise en oeuvre l'utilisation de gaz propulseurs ou d'aérosols. Ainsi les mousses dites aérosol ou spray sont exclues du champ de l'invention. De même, les compositions moussantes rinçables de l'art antérieur de type compositions moussantes classiques riches en tensioactifs moussants et/ou les formules moussantes à teneur plus réduites en tensioactifs mais nécessitant un système mécanique générateur de mousse (type pulvorex) sont également exclues de l'invention. Il en va de même des compositions moussantes faisant intervenir un procédé de foisonnage.

La présente invention a enfin pour objet un médicament destiné à une application topique sur la peau, comprenant une telle composition.

Lé présente invention a également pour objet la composition selon l'invention, pour son utilisation dans le traitement du psoriasis.

La présente invention sera décrite plus en détail dans la description et les exemples ci-après.

La composition selon l'invention est capable de prendre la forme d'une mousse par le seul fait de sa composition et peut ainsi être également définie comme une composition auto-moussante pour application topique.

La présente invention a, en conséquence, pour premier objet une composition contenant du propionate de clobétasol destinée à une application topique non rincée se présentant sous la forme d'une mousse, avantageusement de consistance semi-solide, ne contenant avantageusement pas de tensioactif moussant, et comprenant un milieu pharmaceutiquement compatible avec une application topique non rincée notamment sur la peau et les phanères.

Par composition se présentant sous la forme d'une mousse, (également dénommée ci-après composition auto-moussante), il est entendu une composition de consistance semi-solide ayant une forme aérée assimilable à une mousse.

La composition auto-moussante selon la présente invention comprend au moins deux compositions ou formules intermédiaires :

- au moins une composition intermédiaire B comprenant un agent générateur de gaz et au moins un agent gélifiant et/ou agent de suspensions choisi parmi les polymères d'acide acrylique, les polysaccharides, la famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspensions choisi parmi les polysaccharides, la famille des aluminium magnésium silicates, la famille des amidons modifiés, la famille des carraghénanes et le PVA, et
- du propionate de clobétasol contenu dans l'une au moins desdites formules intermédiaires A et B.

De préférence, le propionate de clobétasol est contenu dans la composition intermédiaire A.

La composition selon l'invention est auto-moussante, c'est-à-dire qu'elle mousse par simple mélange des compositions intermédiaires A et B. L'invention a également pour objet la composition sous forme de mousse résultant du mélange desdites compositions intermédiaires A et B.

En fonction des agents présents dans chaque composition intermédiaire (ou formule intermédiaire), ainsi que de leurs proportions dans ladite composition, celles-ci peuvent se présenter sous toutes les formes galéniques ou

prendre toutes les textures connues utilisables en pharmacie, pour une application topique.

De manière préférée, chaque composition intermédiaire (ou formule intermédiaire) selon l'invention peut donc se présenter par exemple sous forme de gel, émulsion (crème, crème sans tensioactif, lotion, lait ou crème fluide), sérum, solution, suspension, et préférentiellement sous la forme d'émulsion (crème, crème sans tensioactif, lotion, lait ou crème fluide) ou de gel. L'ensemble de ces formulations entrent dans le cadre de l'invention.

Selon l'invention, chaque composition (ou formule) intermédiaire peut présenter une viscosité (mesurée à 25°C et à pression atmosphérique) comprise entre 1cP et 500000 cP, avantageusement entre 500 cP et 350000 cP, mesurée avec une méthode classique de type brookfield RV DV II : Aiguille 6 vit 2.

Selon l'invention, on entend par agent activateur de l'agent générateur de gaz un ingrédient qui, par réaction chimique avec l'agent générateur de gaz, libère un gaz. Préférentiellement, il s'agit d'une réaction acide/base.

Selon l'invention le gaz généré par l'agent générateur de gaz peut être tout gaz physiologiquement compatible et permettant l'obtention d'une mousse, comme par exemple le dioxyde de carbone ($CO_2$) ou l'oxygène ($O_2$). De préférence, le gaz généré à partir de l'agent générateur de gaz est du dioxyde de carbone ($CO_2$).

Selon l'invention, la concentration en gaz pouvant varier, la quantité de bulles dans la composition peut varier et ainsi donner une composition pouvant aller de peu aérée à très fortement aérée.

Ainsi selon l'invention, la composition auto-moussante peut se présenter préférentiellement sous toutes les formes allant de aérée à une mousse très expansée.

La composition selon l'invention est adaptée à une application topique et peut comprendre en outre un milieu physiologiquement acceptable, c'est-à-dire un milieu compatible avec la peau et les phanères. Il s'agit de préférence d'un milieu pharmaceutiquement acceptable.

En outre, la composition peut comprendre tout agent actif susceptible de présenter une activité, éventuellement thérapeutique. Ces agents actifs peuvent être, entre autres, choisis parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

Selon l'invention, la composition sous forme de mousse (c'est-à-dire prête à être appliquée) présente un pH compris entre 2 et 8, préférentiellement entre 3 et 7.

Dans la mesure où la ou les composition(s) (ou formule(s)) intermédiaire(s) nécessite(nt) un stockage en au moins 2 compartiments pour des raisons de stabilité des ingrédients, la présente invention se rapporte soit à un unique contenant compartimenté (chaque compartiment accueillant une formule intermédiaire) et de préférence comprenant 2 ou 3 compartiments, soit à un kit comprenant chaque formule intermédiaire stockée indépendamment l'une de l'autre et physiquement séparées.

Le mélange intime extemporané (directement sur la peau ou sur tout autre support) des formules intermédiaires permet d'obtenir la composition sous forme de mousse selon l'invention.

De manière plus spécifique, la composition (ou formule) intermédiaire A peut se présenter sous forme d'une solution, d'une émulsion (lotion, crème, crème sans émulsionnant, lait, crème fluide) ou gel. Cette composition contient avantageusement l'agent activateur de l'agent générateur de gaz, préférentiellement un acide en quantité suffisante (pouvant se présenter sous forme d'un tampon acide/base à pH acide) qui peut être à titre d'exemple non limitatif le couple acide citrique/citrate de sodium.

La formule B peut se présenter sous forme d'une solution, d'un gel, d'une émulsion (lotion, crème, crème sans émulsionnant, lait, crème fluide). Cette composition contient avantageusement en quantité suffisante un agent générateur de gaz, qui peut être en particulier du bicarbonate de sodium.

Ainsi, l'invention a également pour objet un kit ou un contenant unique à plusieurs compartiments tel que défini précédemment, permettant la préparation extemporanée d'une composition sous forme de mousse selon l'invention, comprenant de manière séparée au moins deux formules intermédiaires (ou compositions intermédiaires):

- une formule intermédiaire A telle que définie ci-avant et comprenant au moins un agent activateur de l'agent générateur de gaz; et
- une formule intermédiaire B telle que définie ci-avant et comprenant au moins un agent générateur de gaz ;
- du propionate de clobétasol étant contenu dans l'une au moins desdites formules intermédiaires A et B.

De préférence, le propionate de clobétasol est contenu dans la composition intermédiaire A.

AGENT ACTIVATEUR DE GAZ :

L'agent activateur de l'agent générateur de gaz (également désigné par « agent activateur de gaz ») est un composé qui réagit avec l'agent générateur de gaz par une réaction chimique (préférentiellement une réaction acido-basique) libérant un gaz.

Il s'agit avantageusement d'un acide, d'un sel de polyacide partiellement salifié ou bien d'une solution tampon d'acide faible et de sa base conjuguée, ou d'un mélange de tels composés.

Selon l'invention le tampon acide/base dudit acide peut être tout tampon acide /base de l'acide faible comme par exemple un tampon acide citrique/citrate de sodium ou encore un tampon acide tartrique/tartrate de sodium. De préférence, nous citerons les alpha-hydroxyacides qui sont des acides faibles ayant préférentiellement un pKa compris entre 2 et 6 tel que l'acide citrique, l'acide tartrique, l'acide malique, l'acide lactique mais aussi l'acide phosphorique et l'acide pyrophosphorique et leurs sels éventuellement partiellement salifiés tels que le disodium pyrophosphate ou le sodium dihydrogénophosphate appelé encore phosphate monosodique.

Préférentiellement selon l'invention, l'agent activateur de gaz est choisi parmi un tampon acide citrique/citrate de sodium seul, l'acide phosphorique, le phosphate de sodium, le disodium pyrophosphate seuls ou en mélange avec le tampon acide citrique/ citrate de sodium.

Selon un mode de réalisation très préféré, l'agent activateur de gaz est un tampon acide citrique/ citrate de sodium seul ou en mélange avec du phosphate sodique et/ou du disodium pyrophosphate.

Dans des compositions pour peaux sensibles ou pour peaux lésées comme les peaux atteint de psoriasis, la teneur en acide citrique/ citrate de sodium est de préférence inférieure ou égale à 2.4% par rapport au poids total de la composition intermédiaire A, de manière à limiter tout risque de picotement. Afin d'améliorer la tolérance et d'éviter la sensation de picotement, de manière préférée, le tampon acide citrique/citrate de sodium est employé en mélange avec le disodium pyrophosphate ou le sodium dihydrogénophosphate.

Selon l'invention, ledit agent activateur de gaz peut être présent dans la formule intermédiaire A en une quantité pouvant aller de 0,001% à 95% en poids par rapport au poids total de la composition intermédiaire A.

AGENT GENERATEUR DE GAZ :

Par agent générateur de gaz, on entend tout agent qui possède la propriété de générer par réaction chimique un gaz. On citera à cet égard tout composé qui, lorsqu'il est mélangé à un acide faible, peut former un gaz par une réaction chimique équivalente à la suivante :

$$NaHCO_3 + RCOOH \rightarrow RCOONa + H_2O + CO_2$$

Selon l'invention, le gaz généré à partir de l'agent générateur de gaz présent dans la composition intermédiaire B est de préférence du gaz carbonique ou dioxyde de carbone ($CO_2$).

Selon l'invention, l'agent générateur de gaz est de préférence choisi parmi le bicarbonate de sodium, le bicarbonate de potassium, le carbonate de sodium, le carbonate de potassium et leurs mélanges.

Préférentiellement selon l'invention, la formule intermédiaire B comprend un agent générateur de gaz carbonique qui de manière particulièrement préférée est du bicarbonate de sodium.

Ledit agent générateur de gaz peut être présent dans la formule intermédiaire B en une quantité allant de 1% à 10%, préférentiellement de 2% à 8% en poids par rapport au poids de la composition intermédiaire B.

Selon l'invention, la formule intermédiaire A présente un pH acide, avantageusement compris entre 1 et 6, et la formule intermédiaire B présente un pH basique, avantageusement compris entre 7 et 12.

Selon l'invention, l'une (ou les) formule(s) intermédiaire(s) comprend le propionate de clobétasol en quantité allant de 0,0001 à 1%, préférentiellement de 0,001 à 0.5%, encore plus préférentiellement de 0.03 à 0.2%, en poids, par rapport au poids de la composition totale.

Dans la présente description, on entend par composition totale ou formule totale la composition du produit sous forme de mousse après le mélange desdites compositions intermédiaires. De façon préférée, le propionate de clobétasol est contenu dans la composition intermédiaire A qui présente avantageusement un pH acide afin d'optimiser sa stabilité.

La formule intermédiaire A, peut se présenter sous toutes les formes galéniques compatibles avec la forme galénique désirée pour la composition finale obtenue par mélange de la formule A avec la formule B, et garantissant une bonne stabilité du propionate de clobétasol La formule intermédiaire B peut se présenter sous toutes les formes galéniques compatibles avec la forme galénique désirée pour la composition finale obtenue par mélange de la formule B avec la formule A. Avantageusement la formule B peut être un gel, une solution, une suspension, une émulsion (crème, crème sans tensioactif, lotion lait, crème fluide), de préférence une émulsion afin d'apporter un maximum d'émollience.

Selon un mode de réalisation de l'invention, l'une des deux formules intermédiaires (ie, la formule intermédiaire A ou la formule intermédiaire B) se présente sous forme d'un gel. Dans ce mode de réalisation, de préférence l'autre formule intermédiaire n'est pas sous forme de gel.

Chaque formule du kit ou du contenant à plusieurs compartiments tel que défini précédemment, conforme à l'invention comprend un milieu physiologiquement acceptable, véhiculant le ou les composés, et choisi de telle sorte que les composés soient aptes à réagir l'un avec l'autre pour former une composition auto-moussante lors du mélange

d'au moins les formules intermédiaires A et B.
Ainsi, le mélange extemporané d'au moins deux formules, par exemple la formule A et la formule B, crée la composition sous forme de mousse selon l'invention.

Lors du mélange des deux formules A et B, l'agent générateur de gaz, tel que le bicarbonate de sodium, peut réagir avec l'agent activateur de gaz tel que l'acide, et donner ainsi notamment le sel correspondant à l'acide, de l'eau et le gaz $CO_2$. C'est ce gaz, emprisonné dans les bulles de la composition, qui crée la mousse qui caractérise la composition auto-moussante de l'invention.
Ainsi par le mélange d'au moins la formule intermédiaire A et la formule intermédiaire B, on obtient la composition mousse dite composition totale selon l'invention.
Dans la composition obtenue après mélange d'au moins les formules A et B, il peut bien entendu rester de l'agent activateur de gaz et/ou de l'agent générateur de gaz n'ayant pas réagi.

Avantageusement, le kit ou le contenant unique à plusieurs compartiments selon l'invention peut être conçu de telle sorte que lors de la préparation de la composition selon l'invention, les formules intermédiaires A et B peuvent être mélangées en un rapport en poids A/B allant de 0,5 à 2, préférentiellement de 0,5 à 1,5, plus préférentiellement proche de 1 (c'est-à-dire de 0,9 à 1,1), et encore plus préférentiellement de 1. Cela signifie que le kit peut être conçu pour libérer simultanément des doses (en poids) des compositions intermédiaires A et B pouvant être en un rapport en poids allant de 2 doses de B pour 1 dose de A à 2 doses de A pour 1 dose de B, de préférence de 2 doses de B pour 1 dose de A à 3 doses de A pour 2 doses de B. Selon un mode de réalisation préféré de l'invention, le kit est conçu pour libérer simultanément 1 dose en poids de A et 1 dose en poids de B.

Selon l'invention le kit peut se présenter sous toute forme compatible avec d'une part une conservation séparée des formules intermédiaires A et B et d'autre part la capacité à réaliser extemporanément le mélange de A et de B.
Par exemple les formules intermédiaires A et B peuvent être conditionnées dans un boitier avec au moins deux compartiments séparés, chacun contenant A ou B.
Selon un autre aspect, le kit peut se présenter sous la forme d'une seringue à au moins deux corps séparés, chacun muni d'un piston, lesdits deux corps contenant les formules respectives A et B, et étant conçus pour libérer simultanément par exercice d'une force sur le piston les doses désirées de A et B.

L'invention concerne également un procédé de préparation d'une composition selon l'invention, caractérisé en ce que pour obtenir la composition sous forme de mousse, on mélange de façon extemporanée au moins une dose de formule intermédiaire A et une dose de formule intermédiaire B du kit telles que définies plus haut, dans des proportions relatives en poids A/B pouvant aller de 0,5 à 2, préférentiellement de 0,5 à 1,5 et plus préférentiellement de 1.

Afin d'obtenir une mousse (composition finale) optimale, les inventeurs ont cherché expérimentalement les teneurs optimales en agent générateur de gaz (de préférence le bicarbonate de sodium) et en agent activateur de gaz (de préférence l'acide citrique et/ou le disodium pyrophosphate et/ou le sodium dihydrogénophosphate).

Ainsi, il a été déterminé expérimentalement que lorsque l'agent activateur de gaz est l'acide citrique, le ratio pondéral acide citrique/bicarbonate de sodium dans la composition totale est compris entre 0,1 et 2 préférentiellement entre 0,5 à 1 et de façon préférée égal à 0,7.
De la même façon, il a été déterminé que lorsque l'agent activateur de gaz est le disodium pyrophosphate, le ratio pondéral disodium pyrophosphate/bicarbonate de sodium dans la composition totale est compris entre 0,5 et 5 préférentiellement entre 1 et 3 et de façon préférée est égal à 2,4.

De la même façon, il a été déterminé que lorsque l'agent activateur de gaz est le sodium dihydrogénophosphate, le ratio pondéral sodium dihydrogénophosphate mono hydrate/bicarbonate de sodium dans la composition totale est compris entre 0,5 et 5 préférentiellement entre 1 et 3 et de façon préférée est égal à 2.

L'exemplification des ratios bicarbonate de sodium/acide citrique, bicarbonate de sodium/pyrophosphate de sodium, bicarbonate de sodium/sodium hydrogénophosphate est décrit dans l'exemple 4.

De façon surprenante, l'association acide citrique/citrate de sodium, disodium pyrophosphate ou sodium dihydrogenophosphate et un système gélifiant compatible avec la forme galénique a permis d'obtenir une formule aux propriétés physico-chimiques très stables.

Une composition est considérée stable physiquement lorsque ses caractéristiques organoleptiques, son pH, sa viscosité et l'homogénéité du propionate de clobétasol n'évoluent pas avec le temps dans différentes conditions de températures : température ambiante (RT, ou TA), 30°C et 40°C.
Selon l'invention, la température ambiante correspond à une température allant de 15°C à 25°C.

Une composition est considérée stable chimiquement lorsque la teneur en principe actif qu'elle contient n'évolue pas avec le temps dans les différentes conditions de températures (RT et 40°C)
Selon l'invention, la composition est considérée stable quand la teneur en propionate de clobétasol (exprimée en poids par rapport au poids de la formule intermédiaire) est incluse dans les spécifications allant de 90% à 110%.

La composition selon l'invention peut comprendre en outre un ou plusieurs agents choisis parmi les agents dispersants, les agents solubilisants, les agents stabilisants, les agents conservateurs, les corps gras, les agents épaississants, les colorants, les parfums, les tensioactifs, les agents gélifiants, les agents complexants, les agents neutralisants, les agents émulsionnants moussants, les agents émulsionnants non moussants, les charges, les agents séquestrants, les agents réducteurs, les masques d'odeur, les agents plastifiants, les agents adoucissants, les agents hydratants, les pigments, les argiles, les charges minérales, les colloïdes minéraux, les polymères, les protéines, les agents nacrants, les cires, les huiles comme par exemple les paraffines, les silicones, des acides gras, des esters solides d'alcool gras ou d'acides gras, des gommes, les agents mouillants.

Des colorants hydrosolubles tels que le FD&C Blue 1 (de formule brute $C_{37}H_{34}N_2Na_2O_9S_3$) et des colorants liposolubles tel que le Rouge Soudan III ou le Red Nil peuvent également être présents dans l'une au moins des compositions intermédiaires. De tels colorants présentent l'avantage de colorer un des intermédiaires de formulation. Cette coloration permet le contrôle du bon mélange des deux intermédiaires de formulation et de mettre en valeur la formation de la mousse.

Bien entendu, l'homme du métier veillera à choisir ce ou ces éventuels adjuvants additionnels et/ou leur quantité de manière telle que les propriétés de (ou des) actif(s) pouvant être ajoutés à la composition selon l'invention ne soient pas, ou substantiellement pas, altérées par l'adjonction envisagée.

AGENTS GELIFIANTS DE LA FORMULE INTERMEDIAIRE CONTENANT L'ACTIVATEUR DE GAZ

La composition intermédiaire A contenant au moins un agent activateur de gaz contient au moins un agent gélifiant et/ou agent de suspension tel que défini dans la revendication 1.

La formulation A pouvant contenir de fortes quantités d'acide et d'électrolytes, elle peut s'avérer être compliquée à stabiliser. La viscosité et le pouvoir suspensif de ces formules sont souvent durs à garantir dans le temps.

A titre d'exemple de gélifiants et/ou agents de suspension résistants à la fois au aux électrolytes et aux pH acides pouvant entrer dans les compositions A selon l'invention, on peut citer les mélanges prêts à l'emploi tels que le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifier, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, la bentonite vendue sous le nom de polargel HV®, la famille des amidons modifiés tels que l'amidon de pomme de terre modifié vendu sous le nom de Structure Solanace®, la famille des carraghénanes en particulier réparties sous quatre grandes familles : κ, λ, β, ω tel que les Viscarin® et les Gelcarin® commercialisés par la société IMCD. Ou encore la Polyvinyl Alcohol connue aussi sous l'abréviation PVA revendu par Merck sous le nom POLYVINYL ALCOHOL 40-88®. De façon préférée, le Vegum K®, le Simulgel 600 PHA® et le Xantural 180® seront utilisés seul ou en association deux à deux ou les trois ensemble.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire A.

AGENTS GELIFIANTS DE LA FORMULE INTERMEDIAIRE CONTENANT LE GENERATEUR DE GAZ

A titre d'exemple de gélifiants et/ou agent de suspension et/ou gélifiants résistants à la fois aux électrolytes et aux pH basiques pouvant entrer dans les compositions intermédiaires B selon l'invention, on peut citer les polymères d'acide acryliques comme l'Acrylates/C10-30 Alkyl Acrylate Crosspolymer tel que les carbomers dits non sensibles aux électrolytes, vendus sous le nom d'Ultrez 20®, d'Ultrez 10®, de Carbopol 1382® ou de Carbopol ETD2020NF®, AQUA SF1® vendus par la Société Lubrizol, le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifier, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et

carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la bentonite vendue sous le nom de polargel HV®, la famille des aluminium magnésium silicates tel que le Veegum K®, Veegum Plus® ou le Veegum Ultra® vendu par la société Vanderbilt, , la famille des amidons modifiés tels que l'amidon de pomme de terre modifié vendu sous le nom de Structure Solanace® ou de Farine de Tapioca connue sous le nom de Naviance Tapioca P® revendu par Akzo Novel ou de la famille des carraghénanes en particulier réparties sous quatre grandes familles : κ, λ, β, ω tel que les Viscarin® et les Gelcarin® commercialisés par la société IMCD. De façon préférée, le Vegum K®, le Simulgel 600 PHA® et le Xantural 180® seront utilisés seul ou en association deux à deux ou les trois ensemble
L'agent gélifiant tel que décrit ci-dessus peut être utilisé aux concentrations préférentielles allant de 0,001% à 15% et, plus préférentiellement, allant de 0,15% à 5% en poids par rapport au poids de la formule intermédiaire B.

AGENTS HUMECTANTS

Parmi les agents humectants et/ou émollients qui ont pour rôle d'hydrater la peau et de faciliter l'application de la formulation, on utilise optionnellement, sans que cette liste soit limitative, des composés tels qu'un polyol miscible à l'eau à la température ambiante (25°C) notamment choisi parmi les sucres (à titre d'exemple le glucose, le lactose), les polyethylene glycol (PEG) (à titre d'exemple le Lutrol E400®), l'urée, les acides aminés (à titre d'exemple la sérine, la citrulline, l'arginine, l'asparagine, l'alanine).
A titre d'agent humectant et/ou émollient préféré, on peut citer la glycérine.
Les agents humectants peuvent être utilisés, seuls ou en association, aux concentrations préférentielles allant de 0,001% à 50 % et, plus préférentiellement, allant de 0.01% à 30% en poids par rapport au poids de la formule totale.

AGENTS CHELATANTS

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tétra-acétique (EDTA) vendu notamment sous le nom Titriplex III® il peut être utilisé aux concentrations préférentielles allant de 0,001% à 1 % et, plus préférentiellement de 0.05% à 0.1% en poids par rapport au poids de la formule totale.

SOLVANT ET AGENT PROPENETRANT

Les compositions A et B peuvent contenir un ou plusieurs agents propénétrants, qui permettent de faciliter la pénétration des principes actifs, et de dissoudre le principe actif présent dans la composition selon l'invention. Plus particulièrement, il est choisi parmi les alcools volatiles en C1-C4, comme l'éthanol ou l'isopropanol pouvant entrer dans la composition jusqu'à 10% en poids de la composition intermédiaire A.
On peut aussi employer des polyols ayant notamment de 2 à 20 atomes de carbones, de préférence ayant de 2 à 10 atomes de carbone, et préférentiellement ayant de 2 à 6 atomes de carbones, tels que le propylène glycol, le butylène glycol, le pentylène glycol, l'hexylène glycol, le dipropylène glycol, le diéthylène glycol, le propylène 1.3 glycol vendu sous le nom de Zemea par la société DuPont Tate & Lyle Bio Products Company, LLC et leurs mélanges. Le glycol préféré est le propylène glycol pouvant être présent dans la composition intermédiaire A jusqu'à 50% ou plus préférentiellement jusqu'à 25% en poids. Ou encore l'agent solvant et propénetrant peut être choisi parmi les éthers de glycol, tels que l'éthoxydiglycol vendu par Gattefossé sous le nom Transcutol HP. Il peut être compris dans la composition A à une teneur allant de 0.1% à 10% et plus préférentiellement entre 0.5 et 2% en poids par rapport au poids de la formule intermédiaire A.

EXCIPIENTS AUX PROPRIETES SPECIFIQUES

La composition selon l'invention peut contenir un ou plusieurs actifs cosmétiques comme par exemple titre non limitatif l'allantoine aux propriétés anti irritantes, le dipotassium glycyrrhizate pour ces propriétés anti inflamatoires, l'acide salicylique et les goudrons de houille aux propriétés kératolytiques, la capsaïcine qui atténue les démangeaisons ou encore le alpha bisabol cicatrisant.

CHARGES ET PARTICULES

Des charges et/ou des particules peuvent être utilisées pour stabiliser et booster la mousse. Certaines d'entre elles ont la propriété particulière de se placer à l'interface eau/air et ainsi stabiliser cette interface. Comme charges, on peut citer le talc, les oxydes de métaux tel que l'oxyde de zinc, le dioxyde de titane TiO2 T2000 vendu par la société Merck sous le nom Eusolex® T-2000, les argiles tel que les laponites®, bentones® ou les bentonites® mais aussi les ethers de cellulose tel que le Methocel K100 LV® commercialisé par la société DOW, les silices telles que l'Aerosil® R972 vendue par la société EVONIK ou la SILICE HDK® H13L vendue par WACKER. Elles peuvent être utilisées aux concentrations allant de 0.01% à 10% en poids par rapport au poids de la formule totale.

AGENT CONSERVATEUR :

On peut citer à titre d'exemple de conservateurs, le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, le benzoate de sodium ou leurs mélanges.

Au titre de système conservateur préféré on peut citer l'association phénoxyéthanol et pentylene glycol ou le benzoate de sodium.

LES HUILES DE LA PHASE GRASSE

La composition selon l'invention peut également comprendre une phase grasse. Cette phase grasse peut être présente dans l'une et/ou l'autre des compositions intermédiaires A et B. La phase grasse peut selon la forme galénique des formules intermédiaires représenter de 0% à 95% en poids par rapport au poids de chaque formule intermédiaire.

La phase grasse de la composition selon l'invention peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive, l'huile de sésame.

Comme huile animale ou leur substitut d'origine végétale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, l'isononyl isononanoate comme le DUB ININ® revendu par les Stearine Dubois, l'isopropyl myristate vendu sous le nom de crodamol IPM par la société CRODA, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglyceride tel que Miglyol 812® vendu par la société Univar. Comme polyisobutene hydrogéné, on peut citer les Parleam® vendus par la société Rossow, le C12-15 alkyl benzoate vendu sous le nom CRODAMOL AB par la société CRODA, l'octyldodecanol ou Eutanol G vendu par la société BASF, l'alcool oléique vendu sous le nom de KOLLICREAM OA par la société BASF, le PPG-11 STEARYL ETHER l'arlamol PS11E vendu par la société CRODA.

Comme huile de silicone, on peut citer une dimethicone comme le produit vendu sous le nom de Q7-9120 Silicone Fluid® de viscosité de 20 est à 12500 est par la société Dow Corning, une cyclomethicone comme le produit vendu sous le nom de ST-Cyclomethicone 5NF® également par la société Dow Corning.

Ces huiles peuvent être présentes, seules ou en association, à des teneurs allant de 0,5 à 50% en poids, et préférentiellement de 2 à 30% en poids, par rapport au poids de la composition totale.

LES CORPS GRAS NON LIQUIDES

La composition selon l'invention, et en particulier la formule intermédiaire B, peut également comprendre des corps gras solides tel que des cires naturelles ou synthétiques, des acides gras tels que l'acide stéarique, des alcools gras tels que le Speziol C18® pharma ou le Speziol C16® vendu par la société Cognis, et des agents de texture de type tribehenate, tel que le Compritol 888® vendu par la société Gattefossé ou les huiles de ricin hydrogénées telles que le Cutina HR® vendu par la société Cognis ou le glyceryl stearate tel que le GELEOL® vendu par la société Gattefosse ou encore le Dc 9045 Elastomer Blend® vendu par la société Dow Corning.

Ces corps gras non liquides peuvent être utilisés seuls ou en mélange de 0 à 30% en poids par rapport au poids de la formule totale. Il a été cependant observé une qualité de mousse exceptionnelle lorsque des alcool gras de formule $CH_3(CH_2)_nOH$ (n est compris entre 11 et 23) sont présents à des teneurs supérieures à 1% en poids par rapport au poids de la formule totale.

EMULSIFIANTS NON IONIQUES

La composition selon l'invention et notamment la formule intermédiaire B peut également comprendre un ou plusieurs émulsifiants non ioniques.

A titre d'émulsifiants préférés on peut citer des émulsifiants hydrophiles de type Glyceryl Stearate (and) PEG-100 Stearate vendu sous le nom Arlacel 165FL® par la société Uniquema, des émulsifiants lipophiles de type Glucate SS® et Glucamate SSE®, Polyoxyethylene (21) Stearyl Ether vendu sous le nom Brij721® par la société Uniquema ou encore dans la meme famille le Brij S2®, le Brij S20®. La self emulsifying Wax vendu par Croda sous le nom de Polawax NF®. On peut citer également des tensioactifs non ioniques présentant une HLB élevée, les esters de sorbitan tels que le POE(20) sorbitan monooléate, vendu sous le nom de « Tween 80® » (HLB=15) ; le POE(20) sorbitan monostéarate vendu sous le nom de « Tween 60® » (HLB=14.9) ; les éthers d'alcools gras tels que le POE (21) stéaryl ether (HLB= 15.5), ou le ceteareth 20 vendu sous le nom de Eumulgin B2 PH® par Cognis (HLB de 15,5, ou des tensioactifs non ioniques de bas HLB, les esters de sorbitan, tels que le monostéarate de sorbitan (vendu sous le nom de Span 60® par Uniquema), les esters de glycérol tels que le monostéarate de glycérol (Cutina GMS® de chez Cognis), les esters de saccharose de bas HLB comme le distéarate de saccharose. Dans un autre mode selon l'invention les tensioactifs utilisables sont les esters de polyglycerol. Il s'agit d'esters d'acides gras polyglycerinés obtenus par condensation de glycerine. Des émulsionnants glycolipidique tel que le Montanov 202® vendu par la société SEPPIC. Certains émulsionnants peuvent être vendus sous forme de mélange tel que les Emulium Kappa® et Emulium Delta® vendu par Gattefosse. Ces Tensio actifs peuvent être utilisés seuls ou en association de façon à ce que le HLB du système soit supérieur à 12 et préférentiellement supérieur à 15.

De tels émulsifiants peuvent être utilisés entre 0.01% et 30 % en poids par rapport au poids de la composition totale, préférentiellement entre 0.1% et 15%, et plus préférentiellement entre 0.5% et 7 %.

Les exemples suivants illustrent l'invention sans en restreindre la portée.

EXEMPLES

EXEMPLE 1 : exemples de formules

Exemple de formules A : compositions intermédiaires contenant l'agent activateur de gaz, formulés à pH acide

Les formules intermédiaires A ont été préparées suivant le procédé suivant :

Etape 1 : A une température supérieure à 60°c, ajouter sous agitation les gélifiants puis le ou les agents activateurs du générateur de gaz à la phase d'eau principale.

Etape 2 : Parallèlement réaliser la phase active en solubilisant le propionate de clobétasole dans l'agent solubilisant/pro-pénétrant

Etape 3 : A une température inférieure à 30°C ajouter la phase active à la phase principale.

Etape 4 : Ajouter les additifs ainsi que les conservateurs, les actifs cosmétiques, les agents chélatants.

Dans les exemples de formules ci-dessous, les quantités sont exprimées par rapport au poids de la formule intermédiaire et non par rapport au poids de la formule totale.

Exemple A1 :

| Dénomination INCI | % en poids |
|---|---|
| EAU | QSP100 |
| GLYCERINE | 8 |
| MAGNESIUM ALUMINIUM SILICATE | 1 |
| GOMME DE XANTHANE | 0.5 |
| ALCOOL CETOSTEARYLIQUE | 3 |

(suite)

| Dénomination INCI | % en poids |
|---|---|
| CYCLOPENT ASILOXANE | 2 |
| CETEARETH-20 | 3 |
| GLYCERYL DIBEHENATE | 3 |
| PPG-11 STEARYL ETHER | 20 |
| PHENOXYETHANOL | 1 |
| PROPIONATE DE CLOBETASOL | 0.1 |
| DISODIUM EDTA | 0.1 |
| ETHOXYDIGLYCOL | 1.2 |
| ACIDE CITRIQUE | 1.75 |
| CITRATE DE SODIUM | 1.3 |
| SODIUM PYROPHOSPHATE | 6 |
| POLOXAMER 124 | 0.2 |

Exemple A2

| Dénomination INCI | % en poids |
|---|---|
| EAU | QSP100 |
| GLYCERINE | 8 |
| MAGNESIUM ALUMINIUM SILICATE | 1 |
| GOMME DE XANTHANE | 0.5 |
| ALCOOL CETOSTEARYLIQUE | 3 |
| CYCLOPENTASILOXANE | 2 |
| CETEARETH-20 | 3 |
| GLYCERYL DIBEHENATE | 3 |
| HUILE MINERALE | 24 |
| PHENOXYETHANOL | 1 |
| PROPIONATE DE CLOBETASOL | 0.1 |
| ETHANOL | 10 |
| PHENOXYETHANOL | 1 |
| DISODIUM EDTA | 0.1 |
| ACIDE CITRIQUE | 3.5 |
| CITRATE DE SODIUM | 2.7 |

Exemple A3

| Dénomination INCI | % en poids |
|---|---|
| EAU | QSP100 |
| GLYCERINE | 8 |
| MAGNESIUM ALUMINIUM SILICATE | 1 |
| GOMME DE XANTHANE | 0.5 |
| ALCOOL CETOSTEARYLIQUE | 3 |
| CYCLOPENTASILOXANE | 2 |
| CETEARETH-20 | 3 |
| GLYCERYL DIBEHENATE | 3 |
| TRIGLYCERIDE CAPRYLIQUE/CAPRIQUE | 24 |
| PHENOXYETHANOL | 1 |
| PROPIONATE DE CLOBETASOL | 0.1 |
| PROPYLENE GLYCOL | 30 |
| PHENOXYETHANOL | 1 |
| DISODIUM EDTA | 0.1 |
| ACIDE CITRIQUE | 1.7 |
| CITRATE DE SODIUM | 1.3 |
| DISODIUM PYROPHOSPHATE | 6 |

Exemple A4

| Dénomination INCI | % en poids |
|---|---|
| EAU | QSP100 |
| GLYCERINE | 8 |
| MAGNESIUM ALUMINIUM SILICATE | 1 |
| GOMME DE XANTHANE | 0.5 |
| PROPIONATE DE CLOBETASOL | 0.1 |
| PROPYLENE GLYCOL | 30 |
| PHENOXYETHANOL | 1 |
| DISODIUM EDTA | 0.1 |
| ACIDE CITRIQUE | 1.7 |
| CITRATE DE SODIUM | 1.3 |
| DISODIUM PYROPHOSPHATE | 6 |

Exemple A5

| Dénomination INCI | % en poids |
|---|---|
| EAU | QSP100 |

(suite)

| Dénomination INCI | % en poids |
|---|---|
| GLYCERINE | 8 |
| MAGNESIUM ALUMINIUM SILICATE | 1 |
| GOMME DE XANTHANE | 0.5 |
| PROPIONATE DE CLOBETASOL | 0.1 |
| ETHANOL | 10 |
| PHENOXYETHANOL | 1 |
| DISODIUM EDTA | 0.1 |
| ACIDE CITRIQUE | 1.7 |
| CITRATE DE SODIUM | 1.3 |
| DISODIUM PYROPHOSPHATE | 6 |

Exemple de formules B : compositions intermédiaires contenant l'agent générateur de gaz, formulés à pH basique

Les formules intermédiaires ont été préparées suivant le procédé suivant :

Etape 1' : A une température supérieure à 60°C, ajouter sous agitation les gélifiants à la phase d'eau principale.

Etape 2' optionnel : Parallèlement chauffer la phase grasse (contenant les huiles, les cires, et les tensio-actifs) à une température supérieure à 60°C.

Etape 3' optionnel : A une température supérieure à 60°C réaliser l'émulsion en ajoutant la phase grasse à la phase principale.

Etape 4' : Ajouter les additifs tel que les conservateurs ou de l'éthanol à une température adaptée à l'additif.

Etape 5' : Neutraliser le mélange.

Etape 6' : A une température inférieure à 40°C ajouter le bicarbonate de sodium

Exemple B1 :

| Dénomination INCI | % en poids |
|---|---|
| EAU | QSP 100 |
| MAGNESIUM ALUMINIUM SILICATE | 2.5 |
| GOMME DE XANTHANE | 0.6 |
| TRIETHANOLAMINE | 1.2 |
| SODIUM HYDROGENO CARBONATE | 5 |
| PHENOXYETHANOL | 0.8 |

Exemple B2 :

| Dénomination INCI | % en poids |
|---|---|
| EAU | QSP 100 |
| MAGNESIUM ALUMINIUM SILICATE | 2.5 |
| GOMME DE XANTHANE | 0.5 |
| CETEARETH-20 | 3 |
| ALCOOL CETOSTEARYLIQUE | 3 |
| GLYCERYL DIBEHENATE | 3 |
| TRIGLYCERIDE CAPRYLIQUE / CAPRIQUE | 10 |
| DL-ALPHA-TOCOPHEROL | 0.05 |
| HUILE MINERALE | 5 |
| HUILE DE NOIX DE COCO (COCOS NUCIFERA | 2 |
| PRUNUS AMYGDALUS DULCIS | 5 |
| CYCLOPENTASILOXANE | 3 |
| HYDROXYDE DE SODIUM | 0.09 |
| PHENOXYETHANOL | 0.8 |
| SODIUM HYDROGENOCARBONATE | 5 |

Exemple B3 :

| Dénomination INCI | % en poids |
|---|---|
| EAU | QSP 100 |
| MAGNESIUM ALUMINIUM SILICATE | 2.5 |
| GOMME DE XANTHANE | 0.5 |
| CETEARETH-20 | 3 |
| ALCOOL CETOSTEARYLIQUE | 3 |
| GLYCERYL DIBEHENATE | 3 |
| TRIGLYCERIDE CAPRYLIQUE / CAPRIQUE | 10 |
| DL-ALPHA-TOCOPHEROL | 0.05 |
| HUILE MINERALE | 2 |
| PRUNUS AMYGDALUS DULCIS | 5 |
| HUILE DE RICIN | 2 |
| DIMETHICONE | 3 |
| HYDROXYDE DE SODIUM | 0.09 |
| PHENOXYETHANOL | 0.8 |
| SODIUM HYDROGENOCARBONATE | 5 |

Exemple B4 :

| Dénomination INCI | % en poids |
|---|---|
| EAU | QSP 100 |
| MAGNESIUM ALUMINIUM SILICATE | 2.5 |
| GOMME DE XANTHANE | 0.5 |
| CETEARETH-20 | 3 |
| ALCOOL CETOSTEARYLIQUE | 3 |
| GLYCERYL DIBEHENATE | 3 |
| TRIGLYCERIDE CAPRYLIQUE / CAPRIQUE | 10 |
| DL-ALPHA- TOCOPHEROL | 0.05 |
| BEURRE DE KARITE | 3 |
| PRUNUS AMYGDALUS DULCIS | 5 |
| DIMETHICONE | 2 |
| HYDROXYDE DE SODIUM | 0.09 |
| PHENOXYETHANOL | 0.8 |
| SODIUM HYDROGENOCARBONATE | 5 |

Exemple B5 :

| Dénomination INCI | % en poids |
|---|---|
| EAU | QSP 100 |
| MAGNESIUM ALUMINIUM SILICATE | 2.5 |
| GOMME DE XANTHANE | 0.5 |
| CETEARETH-20 | 3 |
| ALCOOL CETOSTEARYLIQUE | 3 |
| GLYCERYL DIBEHENATE | 3 |
| TRIGLYCERIDE CAPRYLIQUE/CAPRIQUE | 6 |
| HYDROXYDE DE SODIUM | 0.09 |
| PHENOXYETHANOL | 0.8 |
| SODIUM HYDROGENOCARBONATE | 5 |

Le tableau ci-dessous représente les mélanges dans un ratio pondéral 1:1 des compositions intermédiaires A et B décrites ci-avant. Une croix à l'intersection de deux intermédiaires de formulation indique que le mélange a été testé et a généré une mousse aux propriétés recherchées.

| Formule B / Formule A | B1 | B2 | B3 | B4 | B5 |
|---|---|---|---|---|---|
| A1 | X | X | X | X | X |
| A2 | X | X | X | X | X |
| A3 | X | X | X | X | X |
| A4 | | X | X | X | X |
| A5 | | X | X | X | X |

EXEMPLE 2 : densité de mousse

A partir des exemples de formules décrites dans l'exemple 1, des mesures de densité de mousses ont été réalisées au moment de la mise en contact des 2 formules intermédiaires A et B (T0) puis lorsque la réaction chimique générée par la mise en contact des deux compositions est terminée. Ces études ont montré que la composition finale se présente sous la forme d'une mousse présentant une densité appropriée.

EXEMPLE 3 : Etude comparative de mesure de l'irritation

Protocole d'étude.

Une étude a été réalisée selon le protocole TG439 OCDE en vigueur pour le temps d'application court (temps de contact RHE/produit 15min). Ce protocole a été adapté pour un temps d'application long (temps de contact RHE/produit 18h).

L'objectif de cette étude est d'évaluer la tolérance des supports des formules complètes et intermédiaires sur épidermes humains reconstruits (RHE, modèle Episkin) au travers de :

- l'évaluation de la réduction du MTT (viabilité cellulaire)
- la mesure de la libération d'IL-1alpha (marqueur d'irritation)

Les formules testées sont :

- Une composition intermédiaire de formulation acide placebo (c'est à dire, ne contenant pas de propionate de clobétasol),
- Une composition intermédiaire de formulation basique
- La formule complète composée du mélange de la formulation acide placebo + la formulation basique (dans un ratio 50/50 en poids),
- Une référence commerciale sous forme de produit topique.

Cette étude a démontré que toutes les formules testées sont non irritantes.

EXEMPLE 4 :

Il a été établi de façon empirique la teneur idéale en acide citrique, pyrophosphate de sodium et sodium dihydrogénophosphate monohydrate pour réagir avec 5% de bicarbonate de sodium. Les valeurs sont exprimées en pourcentage poids/poids par rapport au poids de chacune des deux formules intermédiaires.

| | Ratio 1 | Ratio2 | Ratio3 |
|---|---|---|---|
| Bicarbonate de Sodium | 5% | 5% | 5% |
| Acide citrique | 3.5% | - | - |
| Disodium pyrophosphate | - | 12 | - |

(suite)

| | Ratio 1 | Ratio2 | Ratio3 |
|---|---|---|---|
| Sodium Dihydrogénophosphate monohydrate | | - | 7.2% |

Afin que le pH de la formule contenant l'activateur de gaz présente une compatibilité optimale avec la peau, nous avons ajouté du citrate de sodium afin de créer un tampon acide citrique/citrate de sodium.
Une partie du tampon acide citrique/citrate de sodium peut avantageusement être substituée par du disodium pyrophosphate et vice versa comme les teneurs citées à titre d'exemple dans le tableau I ci-dessous :

Tableau III : les valeurs sont exprimées en pourcentage poids/poids par rapport au poids de chacune des deux formules intermédiaires

| | E 1 | E 2 | E 3 | E 4 | E 5 | E 6 | E 7 |
|---|---|---|---|---|---|---|---|
| Bicarbonate de Sodium | 5% | 5% | 5% | 5% | 3% | 3% | 3% |
| Acide citrique | 3.5% | 1.75% | 1.4% | 0 | 2.1% | 1.05% | 0 |
| Citrate de Na | 2.7% | 1.3% | 1% | 0 | 1.6% | 1.15% | 0 |
| Disodium pyrophosphate | 0 | 6% | 7.2% | 12% | 0 | 3.6% | 7.2% |

Une partie du tampon l'acide citrique/citrate de sodium peut avantageusement être substituée par du sodium dihydrogénophosphate mono hydrate et vice versa comme les teneurs citées à titre d'exemple dans le tableau IV ci-dessous :

Tableau IV : les valeurs sont exprimées en pourcentage poids/poids par rapport au poids de chacune des deux formules intermédiaires

| | E 1 | E8 | E9 |
|---|---|---|---|
| Bicarbonate de Sodium | 5% | 5% | 5% |
| Acide citrique | 3.5% | 1.5% | 0 |
| Citrate de Na | 2.7% | 0.5% | 0 |
| Sodium Dihydrogénophosphate monohydrate | 0 | 6.2% | 10% |

Dans un mode particulier, il a été déterminé que lorsque la quantité d'acide citrique est supérieure ou égale à 1,4, la quantité de mousse est optimale lorsque le disodium pyrophosphate est présent dans la composition selon l'équation suivante :

- $$[C]=2.4[B]-2.4[A]/0.7$$

Lorsque :

[C] = teneur en poids en disodium pyrophosphate dans la composition intermédiaire A
[A] = teneur en poids en acide citrique monohydrate dans la composition intermédiaire A
[B] = teneur en poids en bicarbonate de sodium dans la composition intermédiaire B

L'équation ci-dessus permet ainsi de calculer les teneurs optimales entre le bicarbonate de sodium l'acide citrique et le pyrophosphate de sodium.

## Revendications

1. Composition auto-moussante contenant du propionate de clobétasol destinée à une application topique non rincée, comprenant:

   - au moins une composition intermédiaire B comprenant un agent générateur de gaz et au moins un agent gélifiant et/ou agent de suspension choisi parmi les polymères d'acide acrylique, les polysaccharides, la famille des aluminium magnésium silicates, la famille des amidons modifiés et la famille des carraghénanes,
   - au moins une composition intermédiaire A comprenant un agent activateur de l'agent générateur de gaz et au moins un agent gélifiant et/ou agent de suspension choisi parmi les polysaccharides, la famille des aluminium magnésium silicates, la famille des amidons modifiés, la famille des carraghénanes et le PVA, et
   - du propionate de clobétasol contenu dans l'une au moins desdites compositions intermédiaires A et B.

2. Composition selon la revendication précédente, **caractérisée en ce que** le propionate de clobétasol est contenu dans la composition intermédiaire A.

3. Composition selon l'une quelconque des revendications précédentes, **caractérisée en ce qu'**elle ne comprend pas de tensioactifs moussants, et en particulier pas de tensioactifs choisis parmi les tensioactifs anioniques, les tensioactifs cationiques, les tensioactifs amphotères et les tensioactifs non ioniques de la famille des alkylpolyglucosides, et des glucamides.

4. Composition selon l'une quelconque des revendications précédentes, **caractérisée en ce que** l'agent générateur de gaz est choisi parmi le bicarbonate de sodium, le bicarbonate de potassium, le carbonate de sodium, le carbonate de potassium et leurs mélanges, et de préférence l'agent générateur de gaz est le bicarbonate de sodium.

5. Composition selon l'une quelconque des revendications précédentes, **caractérisée en ce que** l'agent générateur de gaz est présent dans la composition intermédiaire B en une quantité allant de 1% à 10%, préférentiellement de 2% à 8% en poids, par rapport au poids de la composition intermédiaire B.

6. Composition selon l'une quelconque des revendications précédentes, **caractérisée en ce que** l'agent activateur de l'agent générateur de gaz est choisi parmi un acide, un sel de polyacide partiellement salifié, une solution tampon d'acide faible et de sa base conjuguée, et les mélanges de ces composés.

7. Composition selon la revendication précédente, **caractérisée en ce que** l'agent activateur de l'agent générateur de gaz est choisi parmi l'acide citrique, l'acide tartrique, l'acide malique, l'acide lactique, l'acide phosphorique et l'acide pyrophosphorique, et les sels de ces acides, et plus préférentiellement ledit agent activateur est choisi parmi :

   - un tampon acide citrique/citrate de sodium seul ;
   - l'acide phosphorique, le phosphate de sodium, le disodium pyrophosphate seuls ou en mélange avec un tampon acide citrique/ citrate de sodium.

8. Composition selon la revendication précédente, **caractérisée en ce que** l'agent activateur de l'agent générateur de gaz est un tampon acide citrique/ citrate de sodium seul ou en mélange avec du phosphate sodique et/ou du disodium pyrophosphate.

9. Composition sous forme de mousse, **caractérisée en ce qu'**elle résulte du mélange desdites compositions intermédiaires A et B selon l'une quelconque des revendications précédentes.

10. Composition selon l'une quelconque des revendications précédentes, **caractérisée en ce qu'**elle contient en outre un ou plusieurs actifs choisi parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

11. Médicament destiné à une application topique sur la peau, comprenant une composition telle que définie dans l'une quelconque des revendications 1 à 10.

12. Composition selon l'une quelconque des revendications 1 à 10, pour son utilisation dans le traitement du psoriasis.

13. Kit ou contenant unique à plusieurs compartiments destiné à une application topique non rincée comprenant de manière séparée au moins deux compositions intermédiaires:

- une composition intermédiaire A comprenant au moins un agent activateur de l'agent générateur de gaz, telle que définie dans l'une quelconque des revendications 1 à 10; et
- une composition intermédiaire B comprenant au moins un agent générateur de gaz telle que définie dans l'une quelconque des revendications 1 à 10;
- du propionate de clobétasol étant contenu dans l'une au moins desdites compositions intermédiaires A et B.

14. Kit ou contenant unique à plusieurs compartiments selon la revendication 13, **caractérisé en ce qu'**il est conçu pour mélanger les compositions intermédiaires A et B en un rapport en poids A/B allant de 0,5 à 2, préférentiellement de 0,5 à 1,5, plus préférentiellement de 0,9 à 1,1, et encore plus préférentiellement de 1.

15. Procédé de préparation d'une composition auto-moussante destinée à une application topique non rincée contenant du propionate de clobétasol, dans lequel on mélange une composition intermédiaire A telle que définie dans l'une quelconque des revendications 1 à 10 avec une composition intermédiaire B telle que définie dans l'une quelconque des revendications 1 à 10, dans des proportions relatives en poids A/B allant de 0,5 à 2, préférentiellement de 0,5 à 1,5 et plus préférentiellement de 1.

## Patentansprüche

1. Selbstschäumende Zusammensetzung, enthaltend Clobetasolpropionat, zur topischen Anwendung ohne Spülen, umfassend:

- mindestens eine Zwischenzusammensetzung B, umfassend ein Gaserzeugungsmittel und mindestens ein Geliermittel und/oder Suspendiermittel, ausgewählt aus Acrylsäurepolymeren, Polysacchariden, der Familie der Aluminiummagnesiumsilikate, der Familie der modifizierten Stärken und der Familie der Carrageene,
- mindestens eine Zwischenzusammensetzung A, umfassend ein Aktivierungsmittel für das Gaserzeugungsmittel und mindestens ein Geliermittel und/oder Suspendiermittel, ausgewählt aus Polysacchariden, der Familie der Aluminiummagnesiumsilikate, der Familie der modifizierten Stärken, der Familie der Carrageene und PVA, und
- Clobetasolpropionat, das in mindestens einer der Zwischenzusammensetzungen A und B enthalten ist.

2. Zusammensetzung nach dem vorhergehenden Anspruch, **dadurch gekennzeichnet, dass** das Clobetasolpropionat in der Zwischenzusammensetzung A enthalten ist.

3. Zusammensetzung nach einem der vorhergehenden Ansprüche, **dadurch gekennzeichnet, dass** sie keine schäumenden Tenside und insbesondere keine Tenside, die aus anionischen Tensiden, kationischen Tensiden, amphoteren Tensiden und nichtionischen Tensiden aus der Familie der Alkylpolyglucoside und der Glucamide ausgewählt sind, umfasst.

4. Zusammensetzung nach einem der vorhergehenden Ansprüche, **dadurch gekennzeichnet, dass** das Gaserzeugungsmittel aus Natriumbicarbonat, Kaliumbicarbonat, Natriumcarbonat, Kaliumcarbonat und ihren Gemischen ausgewählt ist und vorzugsweise das Gaserzeugungsmittel Natriumbicarbonat ist.

5. Zusammensetzung nach einem der vorhergehenden Ansprüche, **dadurch gekennzeichnet, dass** das Gaserzeugungsmittel in der Zwischenzusammensetzung B in einer Menge von 1 bis 10 Gew.-%, bevorzugt von 2 bis 8 Gew.-%, bezogen auf das Gewicht der Zwischenzusammensetzung B, vorliegt.

6. Zusammensetzung nach einem der vorhergehenden Ansprüche, **dadurch gekennzeichnet, dass** das Aktivierungsmittel für das Gaserzeugungsmittel aus einer Säure, einem partiell versalzten Polysäuresalz, einer Pufferlösung einer schwachen Säure und ihrer konjugierten Base und den Gemischen dieser Verbindungen ausgewählt ist.

7. Zusammensetzung nach dem vorhergehenden Anspruch, **dadurch gekennzeichnet, dass** das Aktivierungsmittel für das Gaserzeugungsmittel aus Zitronensäure, Weinsäure, Äpfelsäure, Milchsäure, Phosphorsäure und Pyrophosphorsäure und den Salzen dieser Säuren ausgewählt ist und stärker bevorzugt das Aktivierungsmittel ausgewählt ist aus:

- einem Zitronensäure/Natriumcitrat-Puffer allein,
- Phosphorsäure, Natriumphosphat, Dinatriumpyrophosphat allein oder im Gemisch mit einem Zitronensäure/Natriumcitrat-Puffer.

8. Zusammensetzung nach dem vorhergehenden Anspruch, **dadurch gekennzeichnet, dass** das Aktivierungsmittel für das Gaserzeugungsmittel ein Zitronensäure/Natriumcitrat-Puffer allein oder im Gemisch mit Natriumphosphat und/oder Dinatriumpyrophosphat ist.

9. Zusammensetzung in Schaumform, **dadurch gekennzeichnet, dass** sie aus dem Mischen der Zwischenzusammensetzungen A und B nach einem der vorhergehenden Ansprüche resultiert.

10. Zusammensetzung nach einem der vorhergehenden Ansprüche, **dadurch gekennzeichnet, dass** sie außerdem einen oder mehrere Wirkstoffe, ausgewählt aus Weichmachern, Feuchthaltemitteln, Radikalfängern, entzündungshemmenden Mitteln, Vitaminen, Aufhellungsmitteln, Aknemitteln, anti-seborrhoischen Mitteln, Fungiziden, Keratolytika, Sonnenschutzmitteln, Verschlankungsmitteln, Hautfärbemitteln, enthält.

11. Medikament zur topischen Anwendung auf der Haut, umfassend eine Zusammensetzung nach einem der Ansprüche 1 bis 10.

12. Zusammensetzung nach einem der Ansprüche 1 bis 10 zur Verwendung bei der Behandlung von Psoriasis.

13. Kit oder einzelner Mehrkammerbehälter zur topischen Anwendung ohne Spülen, der getrennt mindestens zwei Zwischenzusammensetzungen umfasst:

- eine Zwischenzusammensetzung A, umfassend mindestens ein Aktivierungsmittel für das Gaserzeugungsmittel, nach einem der Ansprüche 1 bis 10 und
- eine Zwischenzusammensetzung B, umfassend mindestens ein Gaserzeugungsmittel, nach einem der Ansprüche 1 bis 10;
- wobei Clobetasolpropionat in mindestens einer der Zwischenzusammensetzungen A und B enthalten ist.

14. Kit oder einzelner Mehrkammerbehälter nach Anspruch 13, **dadurch gekennzeichnet, dass** es bzw. er zum Mischen der Zwischenzusammensetzungen A und B in einem Gewichtsverhältnis A/B von 0,5 bis 2, bevorzugt von 0,5 bis 1, 5, stärker bevorzugt von 0, 9 bis 1, 1 und noch stärker bevorzugt von 1 gestaltet ist.

15. Verfahren zur Herstellung einer selbstschäumenden Zusammensetzung zur topischen Anwendung ohne Spülen, enthaltend Clobetasolpropionat, wobei man eine Zwischenzusammensetzung A nach einem der Ansprüche 1 bis 10 mit einer Zwischenzusammensetzung B nach einem der Ansprüche 1 bis 10 in jeweiligen Gewichtsanteilen A/B von 0,5 bis 2, bevorzugt von 0,5 bis 1,5 und stärker bevorzugt von 1 mischt.

## Claims

1. Self-foaming composition containing clobetasol propionate, intended for leave-on topical application, comprising:

- at least one intermediate composition B comprising a gas-generating agent and at least one gelling agent and/or suspending agent chosen from acrylic acid polymers, polysaccharides, the family of magnesium aluminium silicates, the family of modified starches and the family of carrageenans,
- at least one intermediate composition A comprising an agent for activating the gas-generating agent and at least one gelling agent and/or suspending agent chosen from polysaccharides, the family of magnesium aluminium silicates, the family of modified starches, the family of carrageenans and PVA, and
- clobetasol propionate contained in at least one of said intermediate compositions A and B.

2. Composition according to the preceding claim, **characterized in that** clobetasol propionate is contained in the intermediate composition A.

3. Composition according to either of the preceding claims, **characterized in that** it does not comprise any foaming surfactants, and in particular no surfactants chosen from anionic surfactants, cationic surfactants, amphoteric surfactants and nonionic surfactants of the family of alkylpolyglucosides and glucamides.

4. Composition according to any one of the preceding claims, **characterized in that** the gas-generating agent is chosen from sodium bicarbonate, potassium bicarbonate, sodium carbonate and potassium carbonate, and mixtures thereof, and preferably the gas-generating agent is sodium bicarbonate.

5. Composition according to any one of the preceding claims, **characterized in that** the gas-generating agent is present in the intermediate composition B in an amount ranging from 1% to 10% by weight and preferentially from 2% to 8% by weight, relative to the weight of the intermediate composition B.

6. Composition according to any one of the preceding claims, **characterized in that** the agent for activating the gas-generating agent is chosen from an acid, a partially salified polyacid salt, a buffer solution of a weak acid and of its conjugate base, and mixtures of these compounds.

7. Composition according to the preceding claim, **characterized in that** the agent for activating the gas-generating agent is chosen from citric acid, tartaric acid, malic acid, lactic acid, phosphoric acid and pyrophosphoric acid, and the salts of these acids, and more preferentially said activating agent is chosen from:

   - a citric acid/sodium citrate buffer alone;
   - phosphoric acid, sodium phosphate, disodium pyrophosphate, which are alone or as a mixture with a citric acid/sodium citrate buffer.

8. Composition according to the preceding claim, **characterized in that** the agent for activating the gas-generating agent is a citric acid/sodium citrate buffer, alone or as a mixture with sodium phosphate and/or disodium pyrophosphate.

9. Composition in foam form, **characterized in that** it results from the mixing of said intermediate compositions A and B according to any one of the preceding claims.

10. Composition according to any one of the preceding claims, **characterized in that** it also contains one or more active agents chosen from emollients, humectants, free-radical scavengers, anti-inflammatory agents, vitamins, depigmenting agents, antiacne agents, antiseborrheic agents, antifungal agents, keratolytic agents, sunscreens, slimming agents and skin-coloring agents.

11. Medicament intended for topical application to the skin, comprising a composition as defined in any one of Claims 1 to 10.

12. Composition according to any one of Claims 1 to 10, for its use in the treatment of psoriasis.

13. Kit or single multi-compartment container, intended for leave-on topical application, separately comprising at least two intermediate compositions:

   - an intermediate composition A comprising at least one agent for activating the gas-generating agent, as defined in any one of Claims 1 to 10; and
   - an intermediate composition B comprising at least one gas-generating agent as defined in any one of Claims 1 to 10;
   - clobetasol propionate being contained in at least one of said intermediate compositions A and B.

14. Kit or single multi-compartment container according to Claim 13, **characterized in that** it is designed for mixing the intermediate compositions A and B in an A/B weight ratio ranging from 0.5 to 2, preferentially from 0.5 to 1.5, more preferentially from 0.9 to 1.1 and even more preferentially 1.

15. Process for preparing a self-foaming composition intended for leave-on topical application, containing clobetasol propionate, in which an intermediate composition A as defined in any one of Claims 1 to 10 is mixed with an intermediate composition B as defined in any one of Claims 1 to 10, in relative weight proportions A/B ranging from 0.5 to 2, preferentially from 0.5 to 1.5 and more preferentially 1.